# EUROPEAN PATENT SPECIFICATION

(11) **EP 2 698 147 B1**
(45) Date of publication and mention of the grant of the patent: **22.07.2015**
(21) Application number: 13180694.5
(22) Date of filing: 16.08.2013
(51) Int. Cl.: A61K 9/00, A61K 31/138, A61K 31/4985, A61K 9/68

(54) **Oral Film Formulations Comprising Dapoxetine and Tadalafil**
Orale Filmformulierungen mit Dapoxetin und Tadalafil
Formulation de film oral comprenant du diapoxetine et tadalafil

(30) Priority: 17.08.2012 TR 201209599; 07.11.2012 TR 201212850
(43) Date of publication of application: 19.02.2014
(73) Proprietor: Sanovel Ilac Sanayi ve Ticaret A.S., 34460 Istanbul (TR)
(72) Inventor: Türkylmaz, Ali, 34460 Istanbul (TR); Yelken, Gülay, 34460 Istanbul (TR)
(74) Representative: Sevinç, Erkan

(56) References cited:
- EP-A1- 2 316 435
- WO-A1-2007/002125
- WO-A2-2008/146178
- DRESSER M J ET AL: "Dapoxetine, a novel treatment for premature ejaculation, does not have pharmacokinetic interactions with phosphodiesterase-5 inhibitors", INTERNATIONAL JOURNAL OF IMPOTENCE RESEARCH, STOCKTON, BASINGSTOKE, GB, vol. 18, no. 1, 1 January 2006 (2006-01-01), pages 104-110, XP009130077, ISSN: 0955-9930
- DATABASE WPI Week 200872 Thomson Scientific, London, GB; AN 2008-M14305 XP002717734, & CN 101 143 145 A (UNIV HEILONGJIANG) 19 March 2008 (2008-03-19)
- "Tadapox-Tadalafil & Dapoxetine Tablet", INTERNET CITATION, December 2010 (2010-12), pages 1-2, XP002702895, Retrieved from the Internet: URL:http://trade.indiamart.com/details.mp? offer=2091554055 [retrieved on 2013-07-15]
- "T-Ject 60 is the newest brand medication. Tadalafil and Dapoxetine", INTERNET CITATION, 23 February 2012 (2012-02-23), pages 1-5, XP002702896, Retrieved from the Internet: URL:http://pills-impotence.com/t-ject-60-i s-the-newest-brand-medication-tadalafil-an d-dapoxetine/ [retrieved on 2013-07-15]

## Description

### Technical Field

The present invention relates to a formulation comprising a combination of dapoxetine or a pharmaceutically acceptable salt thereof and tadalafil or a pharmaceutically acceptable salt thereof. The present invention also relates to a process for preparing this formulation and to the use thereof in the treatment of the premature ejaculation related to the erectile dysfunction.

### Background of Invention

Selective serotonin reuptake inhibitors (SSRI) are used in the long-term prophylaxis of many types of depression, including the endogenous type, recurrent depression, and in the treatment of obsessive-compulsive disorders, panic attack, social phobias, and the bulimia nervosa disease. Dapoxetine, which was first disclosed in the European patent publication EP 0288188 B1 is a selective serotonin reuptake inhibitor. Dapoxetine is used for the treatment of the depression and the premature ejaculation and has the chemical structure shown in Formula I. Additionally, dapoxetine was approved in Switzerland and in Finland for use in the treatment of the premature ejaculation.

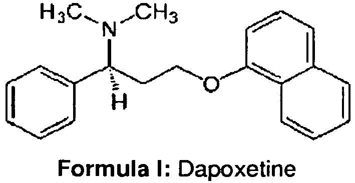

Following oral administration, dapoxetine is rapidly absorbed and rapidly enters the blood circulation by almost completely binding to the plasma proteins. Therefore, it achieves the peak plasma concentration (Cmax) in 1 hour following oral administration. Orally-administered tablets of dapoxetine are commercially available under the name Priligy^{®}, comprising 30 mg or 60 mg dapoxetine hydrochloride per tablet, as well as excipients including lactose monohydrate, microcrystalline cellulose, croscarmellose sodium, colloidal anhydrous silica, magnesium stearate, hypromellose, titanium dioxide (E171), triacetin, black iron oxide (E172) and yellow iron oxide.

The most frequently encountered problem in the oral dapoxetine formulations is the bitter taste thereof. The tablets have typically been coated with the coating agents, and the mixtures of sweeteners or cation exchange resins have been used for masking the bitter taste.

On the other hand, phosphodiesterase type 5 inhibitors (PDE5 inhibitor) are used in the treatment of erectile dysfunction (ED). PDE5 inhibitors block the phosphodiesterase enzyme in a selective and efficient manner, thus increasing the level of cyclic guanosine monophosphate (cGMP) in the corpus cavernosum smooth muscle cells. Most frequently used PDE5 inhibitors are avanafil, lodenafil, mirodenafil, sildenafil, tadalafil, vardenafil, and udenafil. Tadalafil is a PDE5 inhibitor used in the treatment of ED and pulmonary arterial hypertension (PAH). It has a longer half life as compared to other PDE5 inhibitors (mean, 17,5 hours). The chemical designation of tadalafil is (6R-trans)-6-(1,3-benzodioxol-5-yl)- 2,3,6,7,12,12a-hexahydro-2-methyl-pyrazino [1',2':1,6] pyrido[3,4-b]indole-1,4-dione, with the chemical structure illustrated below in Formula II.

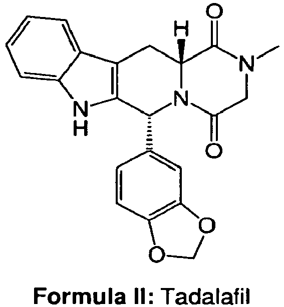

The formulations comprising the combination of selective serotonin reuptake inhibitors with PDE5 inhibitors are known in the prior art. The patent publication WO03000343, for example, discloses the use of the formulation comprising phosphodiesterase inhibitors and dapoxetine. On the other hand, MJ. Dresser et al. stated that dapoxetine, a selective serotonin reuptake inhibitor, does not have pharmaceutical interactions with PDE5 inhibitors and may be used for the treatment of the premature ejaculation (Int J Impot Res. 2006 Jan-Feb;18(1):104-10).

Various formulations and methods for preparing oral film formulations are already known. However, concerning oral administration, oral film formulations have become an issue with increasing importance in terms of patient compliance as compared to conventional solid dosage forms such as capsules and tablets. This issue is more important in terms of patients having difficulty in swallowing. Additionally, concerning many drugs, the swallowing of the same usually necessitates fluids to increase the gastric volume and increases the likelihood of nausea and vomiting. Probably the greatest advantage of an oral film dosage form is that the solid dosage forms rapidly dissolve or disintegrate in the oral cavity so that a solution or suspension forms therein without having to take any liquid. Additionally, the oral films having a low layer thickness and therefore a large surface area rapidly disintegrate in the mouth mucosa. Thus, it will suffice to administer the dosage form briefly before the patient will need it. In addition to these, the oral film dosage forms are more easily used and carried. They are preferred by patients since they do not require carrying any blister packs. Additionally, since tadalafil which is poorly soluble in water is similarly poorly dissolved in gastric juice, its absorption in the stomach is low. For these reasons, the oral film dosage forms are one of the advantageous ways for administering drugs such as dapoxetine and tadalafil and provide a better patient compliance along with the recommended pharmaceutical therapies.

Apart from that, the oral film dosage forms are one of the advantageous ways for administering these drugs to such patients. The oral film formulations provide for a more rapid absorption of the drug in the buccal mucosa, and this may reduce the first pass effect and thus the efficiency of the drug is enhanced. Since this dosage form stays away from the hepatic first-pass metabolism, it increases the clinic effects of some drugs by increasing the bioavailability and decreasing the side-effects thereof. Additionally, since the oral disintegration time thereof is lower as compared to the orally-disintegrating tablet formulations, they are more rapidly absorbed by the mucosa.

Developing an oral film composition is known to be difficult for several different reasons. First, the oral film formulations comprising an acid source may be problematic based on their unpleasant taste. Moreover, these compositions should be flexible and not too rigid. These compositions are quite susceptible to the moisture and the films are prone to adhere to each other. As a result of this, they may show some stability problems.

In order to fulfill all these requirements described above, a special drug formulation is needed and therefore the excipients should be selected carefully. The selected excipients, however, may give formulations with improper bioavailability as compared to equivalent conventional dosage forms. For this reason, the excipients should be selected very carefully. For instance, in the patent application WO2007/002125 is used an orally-disintegrating carrier for providing a rapid oral disintegration of the formulation comprising a PDE5 inhibitor and a SSRI. However, no oral film formulation was disclosed previously which comprises the combination of dapoxetine and tadalafil.

Therefore, the oral film composition of dapoxetine and tadalafil and a process for preparing this composition are required. Other advantages and embodiments of the present invention will be clarified in the following description.

### Detailed Description of Invention

The main object of the present invention is to provide an improved oral film composition of the combination of dapoxetine or a pharmaceutically acceptable salt thereof and tadalafil or a pharmaceutically acceptable salt thereof by making use of convenient excipients, which overcomes the problems mentioned above and is useful in the treatment of the premature ejaculation and the related symptoms thereof.

Another object of the present invention is to provide a process for preparing an improved oral film composition of a combination of dapoxetine or a pharmaceutically acceptable salt thereof and tadalafil or a pharmaceutically acceptable salt thereof.

In one embodiment, the amount of dapoxetine or a pharmaceutically acceptable salt thereof is 5.0 to 30% by weight and of the amount of tadalafil or a pharmaceutically acceptable salt thereof is 5.0 to 30% by weight in the oral film composition.

A further object of the present invention is to provide stable oral film dosage forms, which do not stick to each other, and have a taste which is not unpleasant, comprising a combination of dapoxetine or a pharmaceutically acceptable salt thereof and tadalafil or a pharmaceutically acceptable salt thereof. According to this object, unexpected advantages are found with an oral film composition according to the invention, as compared to currently-available conventional solid dosage forms.

In the orally-disintegrating oral film compositions according to the present invention, suitable amounts of suitable excipients have to be used which do not adhere to each other during storage and therefore are stable throughout the shelf life, and prevent the bitter taste. The selection of the sweetener or the mixture of sweeteners from the group comprising thaumatin, mogroside, sucralose, erythritol, inulin, fructose and the mixtures thereof has surprisingly provided the oral film dosage forms which does not stick to each other and have a taste which is not unpleasant.

According to an embodiment, it was observed that a proper adjustment of the amount of the sweetener made the oral film composition stable throughout the shelf life and prevented the adherence of oral film dosage forms to each other. Accordingly, setting the amount of the sweetener or of the sweetener mixture from 0.01 to 20.0%, preferably from 0.1 to 15.0% and more preferably from 1.0 to 10.0% of the total weight of the composition has avoided the occurrence the aforesaid problems.

It was surprisingly observed that the use of a stability-enhancing agent beside the sweetener in the oral film formulation, comprising dapoxetine or a pharmaceutically acceptable salt thereof and tadalafil or a pharmaceutically acceptable salt thereof, increased the stability of the oral film formulation and thus contributed to obtaining the oral film composition which is stable throughout the shelf life, as well as positively influenced the mechanical resistance of the formulation.

According to an embodiment, the amount of the stability-enhancing agent is from 0.001 to 20.0%, preferably from 0.05 to 15.0%, and more preferably from 0.1 to 10.0% of the total weight of the oral film composition, wherein these amounts provide for a significant increase in the stability of the oral film composition. Amounts below those indicated negatively influence the integrity of the oral film composition and make it prone to disintegration.

According to this embodiment of the present invention, keeping the ratio of the total weight of the sweeteners to the weight of the stability-enhancing agent in the oral film composition between 100:1 to 1:50, preferably between 50:1 to 1:10, and more preferably between 10:1 to 1:1 positively influences the stability of the oral film composition.

According to another embodiment, preferably mogroside is used as the sweetener. Mogroside is a natural sweetener and is used in many fields. Mogroside, which is 300-400 times sweeter than sucrose is used in diabetic foodstuffs. Studies conducted have shown that mogroside, which is composed of proteins bound by disulfide bonds, is not toxic to human body. Mogroside masks the bitter taste by providing a sweet taste without increasing the blood sugar and provides an aroma enhancing effect. In addition to these, since the melting point of the mogroside sweetener is below 190°C, the oral film dosage formulations comprising mogroside do not stick to each other as compared to the oral film dosage formulations comprising sucrose. Thus, the oral film dosage forms can be obtained which are stable throughout the shelf life. Additionally, since mogroside does not increase the blood sugar, it does not cause weight gain and may be used by diabetic patients. Thus, the patient compliance can be increased.

According to a further embodiment, preferably glutathione or tocopherol is used as the stability-enhancing agent.

According to another object of the present invention, the thickness of the oral films obtained influences the flexibility and the friability of the oral films. Accordingly, the thickness of the oral film is between 0.001 and 3 mm, preferably between 0.003 and 1.1 mm, and more preferably between 0.004 and 1 mm.

In addition to these active agents, the sweetener, and the stability-enhancing agent, the oral film composition according to the present invention further comprises at least one pharmaceutically acceptable excipient which is selected from the group comprising of plasticizers, fillers, acid sources, polymers, pH regulating agents, aromatic agents, and colorants.

It was unexpectedly found that the use of a plasticizer in addition to the sweetener or the mixture of sweeteners and stability-enhancing agents in the oral film formulations containing dapoxetine or a pharmaceutically acceptable salt thereof and tadalafil or a pharmaceutically acceptable salt thereof has a synergistic effect on the oral film dosage forms in that they can be stored in a storage container without sticking to each other. Suitable plasticizers are polyethylene glycol or dibutylphtalate. According to this object, the oral film composition according to the present invention comprises the plasticizer in an amount from 0.1 to 30.0% and preferably from 1 to 20.0% based on the total weight of the composition.

Suitable fillers include, but are not limited to sugars, mannitol, sorbitol, sucrose, inorganic salts, calcium salts, polysaccharides, dextrose, dicalsium phosphate, sodium chloride, dextrates, lactitol, maltodextrin, sucrose-maltodextrin mixtures, xylitol, trehalose, heavy magnesium carbonate, and the mixtures thereof. The oral film formulation according to the present invention comprises a filler in an amount from 5.0 to 50.0% and preferably from 10.0 to 40.0% based on the total weight of the composition.

Suitable acid sources include, but are not limited to citric acid, tartaric acid, ascorbic acid, fumaric acid, malic acid; amino acid hydrochlorides, sodium dihydrogen citrate, disodium hydrogen citrate, sodium acid phosphate and similar acid salts; nicotinic acid, acetylsalicylic acid, adipic acid, and the mixtures thereof. The oral film formulation according to the present invention comprises an acid source in an amount from 0.1 to 10.0% and preferably from 1 to 5.0% based on the total weight of the composition. Suitable polymers include, but are not limited to poloxamer, polyacrylamide, polyvinyl alcohol and similar synthetic polymers, hydroxypropyl cellulose, ethyl cellulose, hydroxypropyl methylcellulose, methyl cellulose and similar semi-synthetic polymers; polymethacrylates, polyvinyl acetate, cellulose acetate phthalate, ethylene vinyl acetate, methyl aminoethyl methacrylate, neutral methacrylic acid esters, polyactide, polyactide covinyl glycolide, low molecular weight polyethylene/polyisobutylene, polyanhydrates, diethyl aminoethyl methacrylate, and the mixtures thereof. The oral film formulation according to the present invention comprises a polymer or a mixture of polymers in an amount from 0.1 to 60.0% and preferably from 1 to 50.0% based on the total weight of the composition.

Suitable pH regulating agents include, but are not limited to aluminum potassium sulfate, anhydrous citric acid, anhydrous disodium, hydrogen phosphate, anhydrous sodium dihydrogen phosphate, citric acid, dibasic potassium sulfate, dry sodium carbonate, diluted hydrochloric acid, glacial acetic acid, lactic acid, maleic acid, monobasic potassium phosphate, phosphoric acid, sodium acetate, sodium bicarbonate, sodium carbonate, sodium citrate, sodium dihydrogen phosphate dihydrate, tartaric acid, tribasic sodium phosphate, and the mixtures thereof. The oral film formulation according to the present invention comprises a pH regulating agent in an amount from 0.1 to 10.0% based on the total weight of the composition

Suitable aromatic agents include, but are not limited to fruit aromas like those of orange, banana, strawberry, cherry, wild cherry, lemon, etc., and cardamom, anis, mint, menthol, vanillin, and ethyl vanillin, and other aromas, and the mixtures thereof. The oral film formulation according to the present invention comprises an aromatic agent in an amount from 0.1 to 6.0% based on the total weight of the composition.

Suitable colorants include, but are not limited to, food, drug, and cosmetic (FD&C) dyes (e.g. FD&C blue, FD&C green, FD&C red, FD&C yellow, FD&C lake), ponceau, indigo drug & cosmetic (D&C) blue, indigotine FD&C blue, carmoisine indigotine (indigo carmine); iron oxides (e.g. iron oxide red, yellow, black), quinoline yellow, flame red, brilliant red (carmine), carmoisine, sunset yellow, etc., and the mixtures thereof.

In another aspect, the present invention shows the possibility of providing significant influences on the disintegration time of the oral film dosage form by modifying the size and the shape thereof. In general terms, the disintegration process of an oral film dosage form occurs after the entire surface of an oral film dosage form gets wet by means of capillary effect. Additionally, all shapes maximizing the contact surface with saliva may provide a significant reduction in the disintegration time.

A preferred shape of the oral film composition according to the present invention is a disk, circle, ellipse, triangle, square, polygon, sphere, bar, etc..

A formulation according to the present invention may be produced by means of solvent casting method, semi-solid casting method, solid dispersion extrusion method, rolling method, hot melt extrusion method.

In the solvent casting method, polymer(s) in a formulation are dissolved in a suitable solvent to give a solution. The active agents and other excipients in the formulation are dissolved in a suitable solvent to give another solution. Then both solutions are mixed and entrapped air is removed under vacuum. The resulting solution is cast to form oral film. Then the film is dried and cut into a desired size.

In the semi-solid casting method, separate solutions of the water-soluble polymer and the acidic polymers, e.g. cellulose acetate phthalate, are prepared in suitable solvents. Both these solutions are mixed and then the other excipients and the active agents are added to this mixture. The resulting mixture is coated onto unprocessed cast oral film. Then the film is dried, and cut into a desired size.

In the solid dispersion extrusion method, a solid dispersion of the active agent is prepared together with other materials in the formulation and then processed in a hot melt extrusion device. The resulting product is turned into the oral films. Then the film is cooled and cut into a desired size.

In the rolling method, the solutions of the active agents and other excipients are prepared in the suitable solvents. Then these solutions are rolled onto a substrate to give oral film. Then the film is dried by means of a roller and brought into a desired shape and size.

In the hot melt extrusion method, the active agents and the polymer are mixed together. Other excipients are added to the mixture and the resulting mixture is stirred. Then, it is taken to a hot melt extrusion device and is subjected to the heat treatment. The resulting product is turned into oral film. Then the film is dried and cut into a desired size.

The most preferred processes among those described above are the solvent casting method and the hot melt extrusion method.

The present invention is described in the following examples in more details. These examples are not limiting the scope of the present invention and should be considered under the light of the foregoing detailed disclosure.

### Examples

### Example 1: The oral film composition comprising dapoxetine and tadalafil

| **Ingredients** | **Amount (%)** |
|---|---|
| Tadalafil | 5.00-30.00 |
| Dapoxetine | 5.00-30.00 |
| Starch | 5.00-50.00 |
| Pullulan | 0.10-60.00 |
| Dibutyl phthalate | 0.10-30.00 |
| Mogroside | 0.01-20.00 |
| Glutathione | 0.001-20.00 |
| Aroma | 0.10-6.00 |
| Citric acid | 0.10-10.00 |

### Production method: The solvent casting method

Pullulan used as the polymer is dissolved in a suitable solvent to give a solution. Dapoxetine, tadalafil, starch, dibutyl phthalate, mogroside, glutathione, aroma and citric acid are dissolved in a suitable solvent and the solution is obtained. Then both solutions are mixed and entrapped air is removed under vacuum. The resulting solution is cast to form a film. Then the film is dried and cut into a desired size.

### Example 2: The oral film composition comprising dapoxetine and tadalafil

| **Ingredients** | **Amount (%)** |
|---|---|
| Tadalafil | 5.00-30.00 |
| Dapoxetine | 5.00-30.00 |
| Starch | 5.00-50.00 |
| Pullulan | 0.10-60.00 |
| Polyethylene glycol | 0.10-30.00 |
| Mogroside | 0.01-20.00 |
| Tocopherol | 0.001-20.00 |
| Aroma | 0.10-6.00 |
| Citric acid | 0.10-10.00 |

### Production method: The hot melt extrusion method

Dapoxetine, tadalafil and pullulan are mixed together. Into this mixture starch, polyethylene glycol, mogroside, tocopherol, aroma and citric acid are added and the resulting mixture is mixed. Then, it is taken to the hot melt extrusion device and is subjected to the heat treatment. The resulting product is turned into a film. Then the film is dried and cut into a desired size.

### Example 3: The oral film composition comprising dapoxetine and tadalafil

| **Ingredients** | **Amount (%)** |
|---|---|
| Tadalafil | 5.00-30.00 |
| Dapoxetine | 5.00-30.00 |
| Polyvinylpyrrolidone | 0.10-60.00 |
| Polyethylene glycol | 0.10-30.00 |
| Mogroside | 0.01-20.00 |
| Tocopherol | 0.00-20.00 |
| Aroma | 0.10-6.00 |
| Citric acid | 0.10-10.00 |

### Production method: The solvent casting method

Polyvinylpyrrolidone is dissolved in a suitable solvent to give a solution. Tadalafil, dapoxetine, polyethylene glycol, mogroside, aroma, citric acid and preferably tocopherol are dissolved in a suitable solvent and the solution is obtained. Then both solutions are mixed and entrapped air is removed under vacuum. The resulting solution is cast to form a film. Then the film is dried and cut into a desired size.

### Example 4: The oral film composition comprising dapoxetine and tadalafil

| **Ingredients** | **Amount (%)** |
|---|---|
| Tadalafil | 5.00-30.00 |
| Dapoxetine | 5.00-30.00 |
| Pullulan | 0.10-60.00 |
| Cellulose acetate phthalate | 0.10-60.00 |
| Dibutyl phthalate | 0.10-30.00 |
| Mogroside | 0.01-20.00 |
| Glutathione | 0.00-20.00 |
| Aroma | 0.10-6.00 |
| Citric acid | 0.10-10.00 |

### Production method: The semi-solid casting method

Separate solutions of pullulan and cellulose acetate phthalate (acidic polymer) are prepared in the suitable solvents. Both these solutions are mixed together. Then, into this mixture dibutyl phthalate, mogroside, aroma, citric acid, dapoxetine, tadalafil and preferably glutathione are added. The resulting mixture is coated onto unprocessed cast oral film. Then the film is dried, and cut into a desired size.

### Example 5: The oral film composition comprising dapoxetine and tadalafil

| **Ingredients** | **Amount (%)** |
|---|---|
| Tadalafil | 5.00-30.00 |
| Dapoxetine | 5.00-30.00 |
| Polyvinyl alcohol | 0.10-60.00 |
| Poloxamer | 0.10-20.00 |
| Dibutyl phthalate | 0.10-30.00 |
| Mogroside | 0.01-20.00 |
| Glutathione | 0.00-20.00 |
| Aroma | 0.10-6.00 |
| Iron oxide | 0.10-6.00 |
| Malic acid | 0.10-10.00 |

### Production method: The solid dispersion extrusion method

A solid dispersion of tadalafil and dapoxetine is prepared together with polyvinyl alcohol, poloxamer, dibutyl phthalate, mogroside, aroma, iron oxide, malic acid and preferably glutathione, and then this dispersion is processed in a hot melt extrusion device, the resulting product is turned into a film. Then the film is cooled and cut into a desired size.

### Example 6: The oral film composition comprising dapoxetine and tadalafil

| **Ingredients** | **Amount (%)** |
|---|---|
| Tadalafil | 5.00-30.00 |
| Dapoxetine | 5.00-30.00 |
| Starch | 5.00-50.00 |
| Pullulan | 0.10-60.00 |
| Polyethylene glycol | 0.10-30.00 |
| Mogroside | 0.01-20.00 |
| Glutathione | 0.00-20.00 |
| Aroma | 0.10-6.00 |
| Citric acid | 0.10-10.00 |

### Production method: Rolling method

Solutions of tadalafil, dapoxetine, starch, pullulan, polyethylene glycol, mogroside, the aroma, citric acid and preferably glutathione are prepared in suitable solvents. Then these solutions are rolled onto a substrate and a film is obtained. The film is dried by means of a roller and brought into a desired shape and size.

## Claims

1. An oral film formulation of dapoxetine or a pharmaceutically acceptable salt thereof and tadalafil or a pharmaceutically acceptable salt thereof, comprising at least one sweetener.

2. The oral film formulation according to claim 1, wherein the sweetener or a mixture of sweeteners is selected from the group comprising of at least one a mixture of thaumatin, mogroside, sucralose, erythritol, inulin, fructose.

3. The oral film formulation according to claim 2, wherein the amount of the sweetener or a mixture of sweeteners is 0.01 to 20.0% by weight, preferably from 0.1 to 15.0% by weight, and more preferably from 1 to 10.0% by weight.

4. The oral film formulation according to claims 1 to 3, further comprising at least one stability-enhancing agent.

5. The oral film formulation according to claim 4, wherein the amount of the stability-enhancing agent is in an amount from 0.001 to 20.0% by weight, preferably 0.05 to 15.0% by weight, and more preferably 0.1 to 10.0% by weight.

6. The oral film formulation according to claims 1 to 5, wherein the ratio of the total weight of the sweetener to the weight of the stability-enhancing agent is in the range of 100:1 to 1:50, preferably 50:1 to 1:10, and more preferably 10:1 to 1:1.

7. The oral film formulation according to claim 2, wherein the sweetener is mogroside.

8. The oral film formulation according to claims 4 and 5, wherein the stability-enhancing agent is glutathione or tocopherol.

9. The oral film formulation according to claim 1, wherein the thickness of the oral film is between 0.001 and 3 mm, preferably between 0.003 and 1.1 mm, and more preferably between 0.004 and 1 mm.

10. The oral film formulation according to claim 1, wherein the amount of dapoxetine or a pharmaceutically acceptable salt thereof is 5.0 to 30.0% by weight and the amount of tadalafil or a pharmaceutically acceptable salt thereof is 5.0 to 30.0% by weight.

11. The oral film formulation according to the claims 1 to 10, further comprising at least one pharmaceutically acceptable excipient which is selected from a group comprising plasticizers, fillers, acid sources, polymers, pH regulating agents, aromatic agents, and colorants.

12. The oral film formulation according to claim 11, wherein the plasticizer is polyethylene glycol or dibutyl phthalate.

13. The oral film formulation according to claim 12, wherein the amount of the plasticizer is 0.1 to 30.0% by weight and preferably 1 to 20.0% by weight.

14. The oral film formulation according to claim 11, wherein the filler is selected from a group comprising sugars, mannitol, sorbitol, sucrose, inorganic salts, calcium salts, polysaccharides, dextrose, dicalsium phosphate, sodium chloride, dextrates, lactitol, maltodextrin, sucrose-maltodextrin mixtures, xylitol, trehalose, heavy magnesium carbonate, and the mixtures thereof.

15. The oral film formulation according to claim 14, wherein the amount of the filler is 5.0 to 50.0% by weight and preferably 10.0 to 40.0% by weight.

16. The oral film formulation according to claim 11, wherein the acid source is selected from a group comprising citric acid, tartaric acid, ascorbic acid, fumaric acid, malic acid; amino acid hydrochlorides, sodium dihydrogen citrate, disodium hydrogen citrate, sodium acid phosphate and similar acid salts; nicotinic acid, acetylsalicylic acid, adipic acid, and the mixtures thereof.

17. The oral film formulation according to claim 16, wherein the acid source is in an amount from 0.1 to 10.0% and preferably from 1.0 to 5.0% based on the total weight of the composition.

18. The oral film formulation according to claim 11, wherein the polymer is selected from a group comprising poloxamer, polyacrylamide, synthetic polymers such as polyvinyl alcohol, semi-synthetic polymers such as hydroxypropyl cellulose, ethyl cellulose, hydroxypropyl methylcellulose, methyl cellulose ; polymethacrylates, polyvinyl acetate, cellulose acetate phthalate, ethylene vinyl acetate, methyl aminoethyl methacrylate, neutral methacrylic acid esters, polyactide, polyactide covinyl glycolide, low molecular weight polyethylene/polyisobutylene, polyanhydrates, diethyl aminoethyl methacrylate, and the mixtures thereof.

19. The oral film formulation according to claim 18, wherein the amount of the polymer is 0.1 to 60.0% by weight, and preferably 1.0 to 50.0% by weight.

20. The oral film formulation according to claim 11, wherein the pH regulating agent is selected from a group comprising aluminum potassium sulfate, anhydrous citric acid, anhydrous disodium, hydrogen phosphate, anhydrous sodium dihydrogen phosphate, citric acid, dibasic potassium sulfate, dry sodium carbonate, diluted hydrochloric acid, glacial acetic acid, lactic acid, maleic acid, monobasic potassium phosphate, phosphoric acid, sodium acetate, sodium bicarbonate, sodium carbonate, sodium citrate, sodium dihydrogen phosphate dihydrate, tartaric acid, tribasic sodium phosphate, and the mixtures thereof.

21. The oral film formulation according to claim 20, wherein the amount of the pH regulating agent is 0.1 to 10.0% by weight.

22. The oral film formulation according to claim 11, wherein the aromatic agent is selected from a group comprising fruit aromas like those of orange, banana, strawberry, cherry, wild cherry, lemon, cardamom, anis, mint, menthol, vanillin, and ethyl vanillin, and the mixtures thereof.

23. The oral film formulation according to claim 22, wherein the amount of the aromatic agent is 0.1 to 6.0% by weight.

24. The oral film formulation according to claim 1, wherein the shape thereof is a disk, circle, ellipse, triangle, square, polygon, sphere, or bar.

25. A method for preparing the oral film formulation of dapoxetine or a pharmaceutically acceptable salt thereof and tadalafil or a pharmaceutically acceptable salt thereof according to any of the preceding claims, comprising a solvent casting method, a semi-solid casting method, a solid dispersion extrusion method, a rolling method, or a hot melt extrusion method, preferably the method is a solvent casting method or a hot melt extrusion method.

## Patentansprüche

1. Orale Filmformulierung von Dapoxetin oder einem pharmazeutisch annehmbaren Salz desselben und Tadalafil oder einem pharmazeutisch annehmbaren Salz desselben, umfassend wenigstens ein Süßungsmittel.

2. Orale Filmformulierung nach Anspruch 1, wobei das Süßungsmittel oder eine Mischung von Süßungsmitteln ausgewählt ist aus einer Gruppe umfassend wenigstens eines aus Thaumatin, Mogrosid, Sucralose, Erythritol, Inulin, Fruktose und Mischungen derselben.

3. Orale Filmformulierung nach Anspruch 2, wobei die Menge des Süßungsmittels oder einer Mischung von Süßungsmitteln 0,01 bis 20,0 Gew.-%, bevorzugt 0,1 bis 15,0 Gew.-%, und bevorzugter 1 bis 10 Gew.-%, ist.

4. Orale Filmformulierung nach den Ansprüchen 1 bis 3, weiter umfassend wenigstens ein stabilitätsverbesserndes Mittel.

5. Orale Filmformulierung nach Anspruch 4, wobei die Menge des stabilitätsverbessernden Mittels in einer Menge von 0,001 bis 20,0 Gew.-%, bevorzugt 0,05 bis 15,0 Gew.-% und bevorzugter 0,1 bis 10,0 Gew.-% ist.

6. Orale Filmformulierung nach den Ansprüchen 1 bis 5, wobei das Verhältnis des Gesamtgewichts des Süßungsmittels zum Gewicht des stabilitätsverbessernden Mittels im Bereich von 100:1 bis 1:50, bevorzugt 50:1 bis 1:10, und bevorzugter 10:1 bis 1:1 ist.

7. Orale Filmformulierung nach Anspruch 2, wobei das Süßungsmittel Mogrosid ist.

8. Orale Filmformulierung nach den Ansprüchen 4 und 5, wobei das stabilitätsverbessernde Mittel Glutathion oder Tocopherol ist.

9. Orale Filmformulierung nach Anspruch 1, wobei die Dicke des oralen Films zwischen 0,001 und 3mm, bevorzugt zwischen 0,003 und 1,1mm, und noch bevorzugter zwischen 0,004 und 1mm, ist.

10. Orale Filmformulierung nach Anspruch 1, wobei die Menge an Dapoxetin oder einem pharmazeutisch annehmbaren Salz desselben 5,0 bis 30,0 Gew.-% und die Menge an Tadalafil oder einem pharmazeutisch annehmbaren Salz desselben 5,0 bis 30,0 Gew.-% ist.

11. Orale Filmformulierung nach den Ansprüchen 1 bis 10, weiter umfassend wenigstens einen pharmazeutisch annehmbaren Arzneistoffträger, der ausgewählt ist aus einer Gruppe umfassend Weichmacher, Füllstoffe, Säurequellen, Polymere, pH-regulierende Mittel, aromatische Mittel und Farbstoffe.

12. Orale Filmformulierung nach Anspruch 11, wobei der Weichmacher Polyethylenglycol oder Dibutylphthalat ist.

13. Orale Filmformulierung nach Anspruch 12, wobei die Menge des Weichmachers 0,1 bis 30,0 Gew.-% und bevorzugt 1 bis 20,0 Gew.-% ist.

14. Orale Filmformulierung nach Anspruch 11, wobei der Füllstoff ausgewählt ist aus einer Gruppe umfassend Zucker, Mannitol, Sorbitol, Sucrose, anorganische Salze, Kalziumsalze, Polysaccharide, Dextrose, Dikalziumphosphat, Natriumchlorid, Dextrate, Lactitol, Maltodextrin, Sucrose-Maltodextrin-Mischungen, Xylitol, Trehalose, schweres Magnesiumcarbonat und die Mischungen derselben.

15. Orale Filmformulierung nach Anspruch 14, wobei die Menge des Füllstoffs 5,0 bis 50,0 Gew.-% und bevorzugt 10,0 bis 40, Gew.-% ist.

16. Orale Filmformulierung nach Anspruch 11, wobei die Säurequelle ausgewählt ist aus einer Gruppe umfassend Citronensäure, Weinsäure, Ascorbinsäure, Fumarsäure, Äpfelsäure; Aminosäurehydrochloride, Natriumdihydrogencitrat, Dinatriumhydrogencitrat, saures Natriumphosphat und ähnliche saure Salze; Nikotinsäure, Acetylsalicylsäure, Adipinsäure und die Mischungen derselben.

17. Orale Filmformulierung nach Anspruch 16, wobei die Säurequelle in einer Menge von 0,1 bis 10,0 % und bevorzugt von 1,0 bis 0,5 %, basierend auf dem Gesamtgewicht der Zusammensetzung, vorliegt.

18. Orale Filmformulierung nach Anspruch 11, wobei das Polymer ausgewählt ist aus einer Gruppe umfassend Poloxamer, Polyacrylamid, synthetische Polymere wie Polyvinylalkohol, halbsynthetische Polymere wie Hydroxypropylcellulose, Ethycellulose, Hydroxypropylmethylcellulose, Methylcellulose; Polymethacrylate, Polyvinylacetat, Celluloseacetatphthalat, Ethylenvinylacetat, Methylaminoethylmethacrylat, neutrale Methacrylsäureester, Polyactid, Polyactidcovinylglycolid, Polyethylen/Polyisobutylen mit niedrigem Molekulargewicht, Polyanhydrate, Diethylaminoethylmethacrylat und die Mischungen derselben.

19. Orale Filmformulierung nach Anspruch 18, wobei die Menge des Polymers 0,1 bis 60,0 Gew.-% und bevorzugt 1,0 bis 50,0 Gew.-% ist.

20. Orale Filmformulierung nach Anspruch 11, wobei das pH-regulierende Mittel ausgewählt ist aus einer Gruppe umfassend Aluminiumkaliumsulfat, wasserfreie Citronensäure, wasserfreies Dinatriumhydrogenphosphat, wasserfreies Natriumdihydrogenphosphat, Citronensäure, dibasisches Kaliumsulfat, trockenes Natriumcarbonat, verdünnte Salzsäure, Eisessigsäure, Milchsäure, Maleinsäure, monobasisches Kaliumphosphat, Phosphorsäure, Natriumacetat, Natriumbicarbonat, Natriumcarbonat, Natriumcitrat, Natriumdihydrogenphosphatdihydrat, Weinsäure, tribasisches Natriumphosphat und die Mischungen derselben.

21. Orale Filmformulierung nach Anspruch 20, wobei die Menge des pH-regulierenden Mittels 0,1 bis 10,0 Gew.-% ist.

22. Orale Filmformulierung nach Anspruch 11, wobei das aromatische Mittel ausgewählt ist aus einer Gruppe umfassend Fruchtaromen wie solche von Orange, Banane, Erdbeere, Kirsche, Wildkirsche, Zitrone, Kardamom, Anis, Minze, Menthol, Vanillin und Ethylvanillin und die Mischungen derselben.

23. Orale Filmformulierung nach Anspruch 22, wobei die Menge des aromatischen Mittels 0,1 bis 6,0 Gew.-% ist.

24. Orale Filmformulierung nach Anspruch 1, wobei die Form derselben eine Scheibe, ein Kreis, eine Ellipse, ein Dreieck, ein Viereck, ein Polygon, eine Kugel oder ein Riegel ist.

25. Verfahren zum Herstellen der oralen Filmformulierung von Dapoxetin oder einem pharmazeutisch annehmbaren Salz desselben und Tadalafil oder einem pharmazeutische annehmbaren Salz desselben gemäß einem der vorangehenden Ansprüche, umfassend ein Lösungsmittelgießverfahren, ein semi-festes Gießverfahren, ein Feststoffdispersionsextrusionsverfahren, ein Walzverfahren oder ein Heißschmelzextrusionsverfahren, wobei das Verfahren eines Lösungsmittelgießverfahrens oder einer Heißschmelzextrusionsverfahrens bevorzugt ist.

## Revendications

1. Formulation de film oral de dapoxétine ou d'un sel pharmaceutiquement acceptable de cette dernière et de tadalafil ou d'un sel pharmaceutiquement acceptable de ce dernier, comprenant au moins un édulcorant.

2. Formulation de film oral selon la revendication 1, dans laquelle l'édulcorant ou un mélange d'édulcorants est choisi parmi les éléments d'un groupe comprenant de la thaumatine, du mogroside, du sucralose, de l'érythritol, de l'inuline, du fructose.

3. Formulation de film oral selon la revendication 2, dans laquelle la quantité d'édulcorant ou d'un mélange d'édulcorants est de 0,01 à 20,0 % en poids, de préférence de 0,1 à 15,0 % en poids, et plus préférentiellement de 1 à 10,0 % en poids.

4. Formulation de film oral selon les revendications 1 à 3, comprenant en outre au moins un agent d'amélioration de la stabilité.

5. Formulation de film oral selon la revendication 4, dans laquelle la quantité d'agent d'amélioration de la stabilité est de 0,001 à 20,0 % en poids, de préférence de 0,05 à 15,0 % en poids, et plus préférentiellement de 0,1 à 10,0 % en poids.

6. Formulation de film oral selon les revendications 1 à 5, dans laquelle le rapport entre le poids total de l'édulcorant et le poids de l'agent d'amélioration de la stabilité est compris dans la plage de 100:1 à 1:50, de préférence 50:1 à 1:10, et plus préférentiellement 10:1 à 1:1.

7. Formulation de film oral selon la revendication 2, dans laquelle l'édulcorant est du mogroside.

8. Formulation de film oral selon les revendications 4 et 5, dans laquelle l'agent d'amélioration de la stabilité est du glutathion ou du tocophérol.

9. Formulation de film oral selon la revendication 1, dans laquelle l'épaisseur du film oral est comprise entre 0,001 et 3 mm, de préférence entre 0,003 et 1,1 mm, et plus préférentiellement entre 0,004 et 1 mm.

10. Formulation de film oral selon la revendication 1, dans laquelle la quantité de dapoxétine ou d'un sel pharmaceutiquement acceptable de cette dernière est de 5,0 à 30,0 % en poids et la quantité de tadalafil ou d'un sel pharmaceutiquement acceptable de ce dernier est de 5,0 à 30,0 % en poids.

11. Formulation de film oral selon les revendications 1 à 10, comprenant en outre au moins un excipient pharmaceutiquement acceptable qui est sélectionné parmi un groupe comprenant des plastifiants, des charges, des sources d'acides, des polymères, des régulateurs de pH, des agents aromatiques et des colorants.

12. Formulation de film oral selon la revendication 11, dans laquelle le plastifiant est du polyéthylène glycol ou du phtalate de dibutyle.

13. Formulation de film oral selon la revendication 12, dans laquelle la quantité de plastifiant est de 0,1 à 30,0 % en poids et de préférence de 1 à 20,0 % en poids.

14. Formulation de film oral selon la revendication 11, dans laquelle la charge est sélectionnée parmi les éléments d'un groupe comprenant des sucres, du mannitol, du sorbitol, du sucrose, des sels inorganiques, des sels de calcium, des polysaccharides, du dextrose, du phosphate dicalcique, du chlorure de sodium, des dextrates, du lactitol, de la maltodextrine, des mélanges de sucrose et de maltodextrine, du xylitol, du tréhalose, du carbonate de magnésium lourd, et les mélanges de ces derniers.

15. Formulation de film oral selon la revendication 14, dans laquelle la quantité de charge est de 5,0 à 50,0 % en poids et de préférence de 10,0 à 40,0 % en poids.

16. Formulation de film oral selon la revendication 11, dans laquelle la source d'acides est sélectionnée parmi les éléments d'un groupe comprenant de l'acide citrique, de l'acide tartrique, de l'acide ascorbique, de l'acide fumarique, de l'acide malique ; des hydrochlorures d'acides aminés, du citrate biacide de sodium, de l'hydrogénocitrate disodique, du phosphate acide de sodium et des sels d'acides similaires ; d'acide nicotinique, d'acide acétylsalicylique, d'acide adipique, et les mélanges de ces derniers.

17. Formulation de film oral selon la revendication 16, dans laquelle la source d'acides est en une quantité de 0,1 à 10,0 % et de préférence de 1,0 à 5,0 % sur la base du poids total de la composition.

18. Formulation de film oral selon la revendication 11, dans laquelle le polymère est sélectionné parmi les éléments d'un groupe comprenant du poloxamère, du polyacrylamide, des polymères synthétiques tels que l'alcool polyvinylique, des polymères semi-synthétiques tels que l'hydroxypropylcellulose, l'éthylcellulose, l'hydroxypropylméthylcellulose, la méthylcellulose ; des polyméthacrylates, de l'acétate de polyvinyle, de l'acétate phtalate de cellulose, de l'éthylène acétate de vinyle, du méthacrylate de méthylaminoéthyle, des esters neutres d'acide méthacrylique, du polyactide, du polyactide covinyl glycolide, du polyéthylène/polyisobutylène à faible poids moléculaire, de polyanhydrates, de méthacrylate de diéthylaminoéthyle, et les mélanges de ces derniers.

19. Formulation de film oral selon la revendication 18, dans laquelle la quantité du polymère est de 0,1 à 60,0 % en poids et de préférence de 1,0 à 50,0 % en poids.

20. Formulation de film oral selon la revendication 11, dans laquelle le régulateur de pH est sélectionné parmi les éléments d'un groupe comprenant du sulfate de potassium d'aluminium, de l'acide citrique anhydre, de sodium anhydre de l'hydrogénophosphate, du dihydrogénophosphate de sodium anhydre, de l'acide citrique, du sulfate de potassium dibasique, du carbonate de sodium sec, de l'acide chlorhydrique dilué, de l'acide acétique glacial, de l'acide lactique, de l'acide maléique, du phosphate de potassium monobasique, de l'acide phosphorique, de l'acétate de sodium, du bicarbonate de sodium, du carbonate de sodium, du citrate de sodium, du dihydrogénophosphate de sodium dihydraté, de l'acide tartrique, du phosphate de sodium tribasique, et les mélanges de ces derniers.

21. Formulation de film oral selon la revendication 20, dans laquelle la quantité de régulateur de pH est de 0,1 à 10,0 % en poids.

22. Formulation de film oral selon la revendication 11, dans laquelle l'agent aromatique est sélectionné parmi les éléments d'un groupe comprenant des arômes de fruits tels que ceux de l'orange, la banane, la fraise, la cerise, la cerise sauvage, le citron, la cardamome, l'anis, la menthe, le menthol, la vanilline, et l'éthylvanilline, et les mélanges de ces derniers.

23. Formulation de film oral selon la revendication 22, dans laquelle la quantité d'agent aromatique est de 0,1 à 6,0% en poids.

24. Formulation de film oral selon la revendication 1, dans laquelle cette dernière a la forme d'un disque, d'un cercle, d'une ellipse, d'un triangle, d'un carré, d'un polygone, d'une sphère ou d'une barre.

25. Procédé d'élaboration d'une formulation de film oral de dapoxétine ou d'un sel pharmaceutiquement acceptable de cette dernière et de tadalafil ou d'un sel pharmaceutiquement acceptable de ce dernier selon l'une quelconque des revendications précédentes, comprenant un procédé de coulage au solvant, un procédé de coulage à l'état semi-solide, un procédé d'extrusion de dispersions solides, un procédé de laminage, ou un procédé d'extrusion à chaud, le procédé étant de préférence un procédé de coulage au solvant ou un procédé d'extrusion à chaud.
